# EUROPEAN PATENT SPECIFICATION

(11) **EP 1 507 489 B1**
(45) Date of publication and mention of the grant of the patent: **04.10.2006**
(21) Application number: 03736614.3
(22) Date of filing: 15.05.2003
(51) Int. Cl.: A61B 19/02

(54) **Low profile sharps container system**
Behältersystem mit niedrigem Profil für scharfe Gegenstände
Système de contenant pour objects acérés, de forme ramassée

(30) Priority: 17.05.2002 US 150573
(43) Date of publication of application: 23.02.2005
(73) Proprietor: Sherwood Services AG, 8201 Schaffhausen (CH)
(72) Inventor: MOATS, Donna, L., Taunton, MA 02780 (US); JAPUNTICH, John, C., Woodstock, IL 60098 (US); KLEIN, Ronald, T., Island Lake, IL 60042 (US); SALZMAN, Jack, Chicago, IL 60655 (US)
(74) Representative: Jones, Colin
(86) International application number: PCT/US2003/015237
(87) International publication number: WO 2003/096921

(56) References cited:
- US-A- 5 387 735
- US-B1- 6 283 909

## Description

### FIELD OF THE INVENTION

This invention relates to a closure for a medical waste receptacle. More specifically, this invention relates to a closure positioned to receive and contain medical waste for safe and efficient disposal.

### BACKGROUND OF THE INVENTION

In hospitals, clinics, and similar medical institutions, contamination continues to be of utmost concern. The prevention of the spread of communicable diseases is a major priority; therefore, disposable, single-use, patient care products have become prevalent. Such items are contaminated, once used, and can readily transmit disease. These items include such devices as hypodermic needles, intravenous needles, razors, scalpel blades, or other sharps - all of which are required to be disposed of at their point of usage under current guidelines of the United States Centers for Disease Control.

Various disposal containers for medical wastes have been proposed for the purpose of preventing an individual from gaining access to contaminated items such as sharps once the wastes have been deposited into the container, and many such disposal containers go far to accomplish this purpose. With hospital and medical office space at a premium, however, a challenge exists to accommodate the disposal of a range of sharps sizes while avoiding a bulky container assembly. In many uses, such container assemblies may be wall-mounted and therefore extend into the space of a room from the wall.

Thus, it is desirable for a container to accommodate sharps ranging from small to large, yet have a profile or depth that does not extend excessively into the room in which it is positioned. For example, some larger sharps requiring disposal include 60 cc syringes with the plunger fully extended, butterfly needles, and angel wing transfer devices. Accommodating such larger sharps as well as smaller sharps, without providing an unduly bulky container assembly, presents a significant challenge.

Accordingly, there is a need for a medical waste disposal system that can accommodate a range of sharps sizes, while maintaining an acceptable profile.

US-A-5 387 735 discloses a disposal container for sharps and a disposal system employing the disposal container. The container comprises a hollow container body having an opening at the top to permit access to the interior of the container body and having a barrier disposed adjacent the opening for restricting access to the interior of the container body. The barrier at least in part comprises a first cowl extending over the opening and a complementary second cowl extending beneath the opening, with the second cowl being offset relative to the first. The container includes a retention for preventing items from being dispensed through the opening from the interior of the container body when the container body is upright. The outer enclosure is shaped to accommodate the inner container, and includes a hood conforming to the first cowl.

### SUMMARY OF THE INVENTION

In accordance with the present invention, there is provided a closure for a medical waste receptacle defining a receptacle opening positioned to receive medical waste, said closure comprising:
a hood positionable adjacent the receptacle opening of the medical waste receptacle, said hood having a limiting surface at least partially defining an access opening facilitating access to an interior of said hood; and
a door mounted at least partially within said interior of said hood, said door being mounted for movement about a pivot axis between an opened position wherein a support surface of said door substantially permits access through said access opening of said hood and a closed position wherein said support surface of said door substantially prevents access through said access opening of said hood;
said door being biased by gravity to rotate from said closed position toward said opened position,
said support surface of said door in said closed position forms an angle at least about 100° with respect to a horizontal plane;
said door has a width and a depth defining said support surface, said door having a width: depth ratio at least about 1.9:1; and
said door and said hood together define said access opening in said opened position and form an access angle being at least about 60°.

Preferred embodiments are set forth in the dependent claims.

### BRIEF DESCRIPTION OF THE DRAWINGS

The invention will be described with reference to the exemplary embodiments illustrated in the figures of which:
Fig. 1 is a right side view of the assembly illustrated in Fig. 1.
Fig. 2 is a detailed view of the assembly illustrated in Fig. 1, with the closure in its closed position.
Fig. 3 is a right side view of the assembly illustrated in Fig. 1, with the closure in its closed position.
Fig. 4 is a detailed view of the assembly illustrated in Fig. 3.
Fig. 5A is a front view of an embodiment of a medical waste receptacle with which a closure according to this invention can be used.
Fig. 5B is a side view of the medical waste receptacle illustrated in Fig. 5A.
Fig. 5C is a top plan view of the medical waste receptacle illustrated in Fig. 5A.
Fig. 6 is a perspective view of an embodiment of a hood component of a closure according to this invention.
Fig. 6A is a front view of the hood illustrated in Fig. 6.
Fig. 6B is a right side view of the hood illustrated in Fig. 6.
Fig. 6C is a top plan view of the hood illustrated in Fig. 6.
Fig. 6D is a cross-sectional right side view of the hood illustrated in Fig. 6.
Fig. 6E is a detailed view of the hood illustrated in Fig. 6D.
Fig. 7 is a perspective view of an embodiment of a lid component of a closure according to this invention.
Fig. 7A is a front view of the lid illustrated in Fig. 7.
Fig. 7B is a cross-sectional left side view of the lid illustrated in Fig. 7.
Fig. 7C is a top plan view of the lid illustrated in Fig. 7.
Fig. 8 is a perspective view of an embodiment of a door component of a closure according to this invention.
Fig. 8A is a front view of the door illustrated in Fig. 8.
Fig. 8B is a right side view of the door illustrated in Fig. 8.
Fig. 8C is a bottom plan view of the door illustrated in Fig. 8.

### DETAILED DESCRIPTION OF THE INVENTION

Preferred features of embodiments of this invention will now be described with reference to the figures. It will be appreciated that the scope of the invention is not limited to the embodiments selected for illustration. Also, it should be noted that the drawings are not rendered to any particular scale or proportion. It is contemplated that any of the configurations and materials described hereafter can be modified within the scope of this invention.

Generally, medical waste disposal systems preferably include a receptacle and some form of a closure assembly that permits the introduction of medical waste into the receptacle while limiting access to the interior of the receptacle. For example, such a medical waste disposal system is disclosed in U.S. Patent No. 5,947,285 to Gaba et al.. The closure of this invention provides a medical waste disposal system adapted to accommodate a range of sharps sizes, while maintaining a low profile (e.g., for wall-mounted assemblies, a limited depth of the container assembly protruding from the wall on which it is mounted).

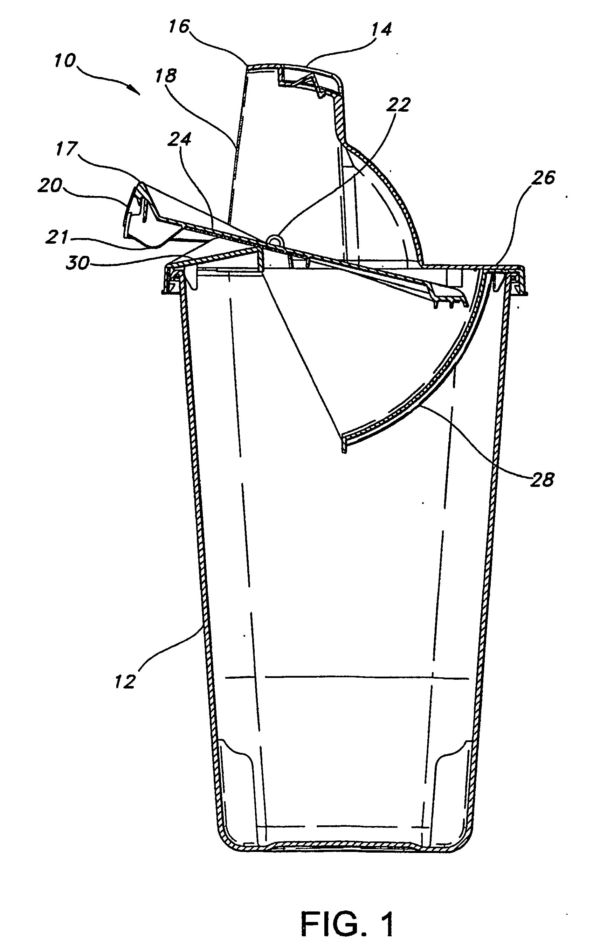

Generally, with reference to Figs. 1 through 8C, this invention provides a closure 10 for a medical waste receptacle 12 defining a receptacle opening 32 positioned to receive medical waste. The closure 10 includes a hood 14 positionable adjacent the receptacle opening 32 of the medical waste receptacle 12. The hood 14 has a hood limiting surface 16 at least partially defining an access opening 18 facilitating access to an interior of the hood 14.

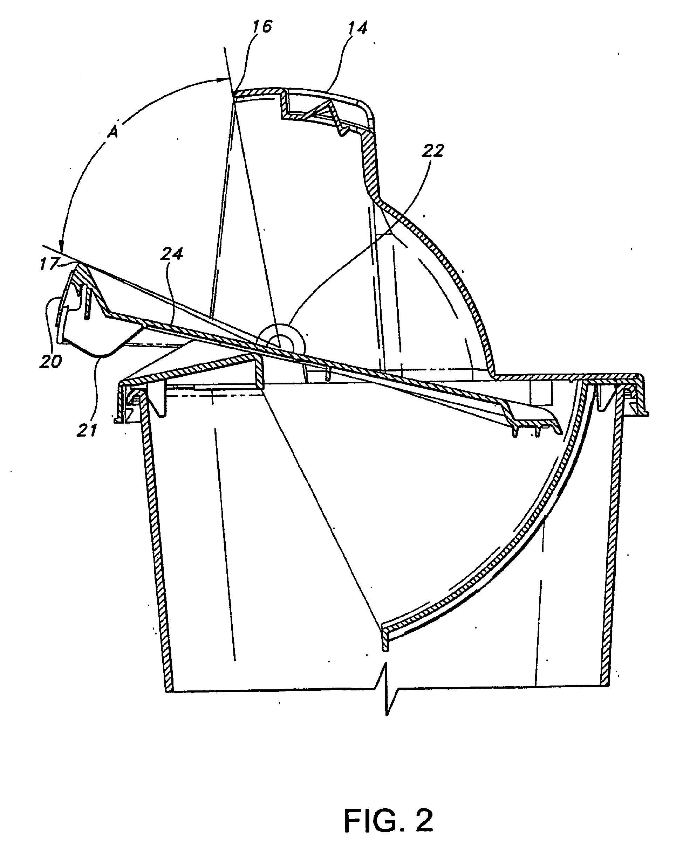

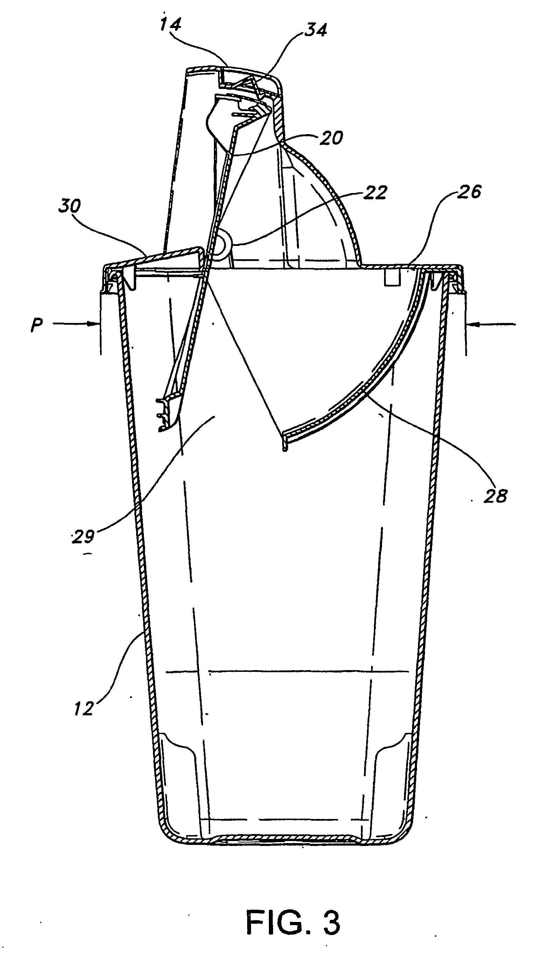

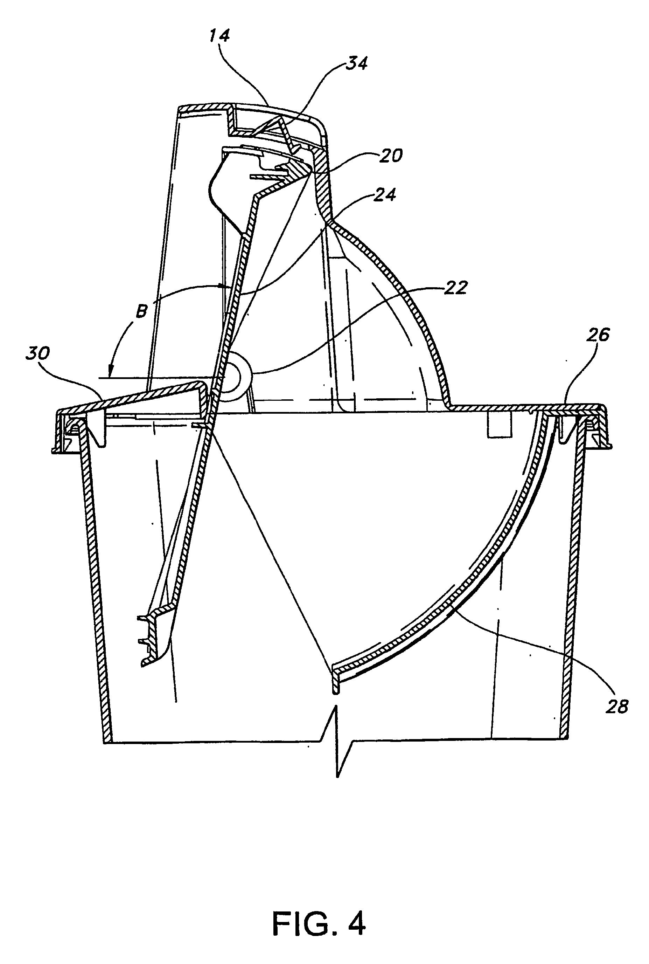

A door 20 is mounted at least partially within the interior of the hood 14. The door 20 is mounted for movement about a pivot axis 22 between an opened position (illustrated in Figs. 1 and 2) and a closed position (illustrated in Figs. 3 and 4). In the opened position, a support surface 24 of the door 20 substantially permits access through the access opening 18 of the hood 14, and in the closed position, the support surface 24 of the door 20 substantially prevents access through the access opening 18 of the hood 14. The door 20 is biased by gravity to rotate from the closed position toward the opened position.

According to one aspect of this invention, the support surface 24 of the door 20 in the closed position forms an angle "B" of at least about 100° with respect to a horizontal plane (see Fig. 4).

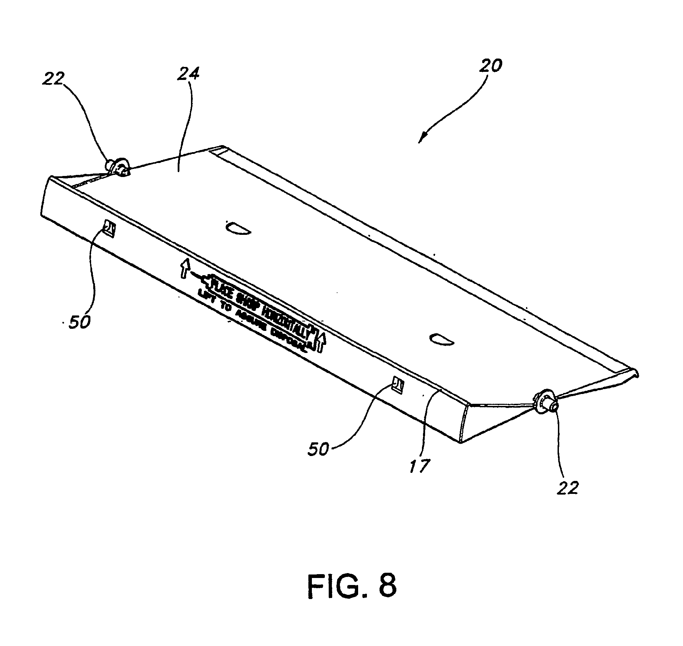

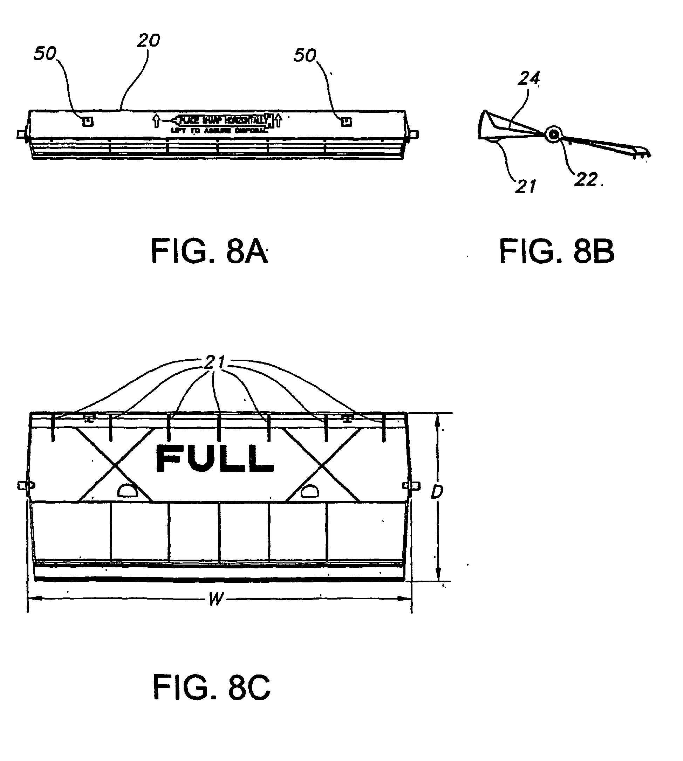

According to another aspect of this invention, the door 20 has a width "W" and a depth "D" defining the support surface 24, and the door 20 has a width:depth ratio of at least about 1.9:1 (see Fig. 8C).

A further aspect of this invention is that the door 20 and the hood 14 together define the access opening 18 in the opened position, forming an access angle "A" (see Fig. 2). According to yet another aspect of this invention, the access angle "A" is defined by the pivot axis 22 of the door 20 and the access opening 18, and is at least about 60°.

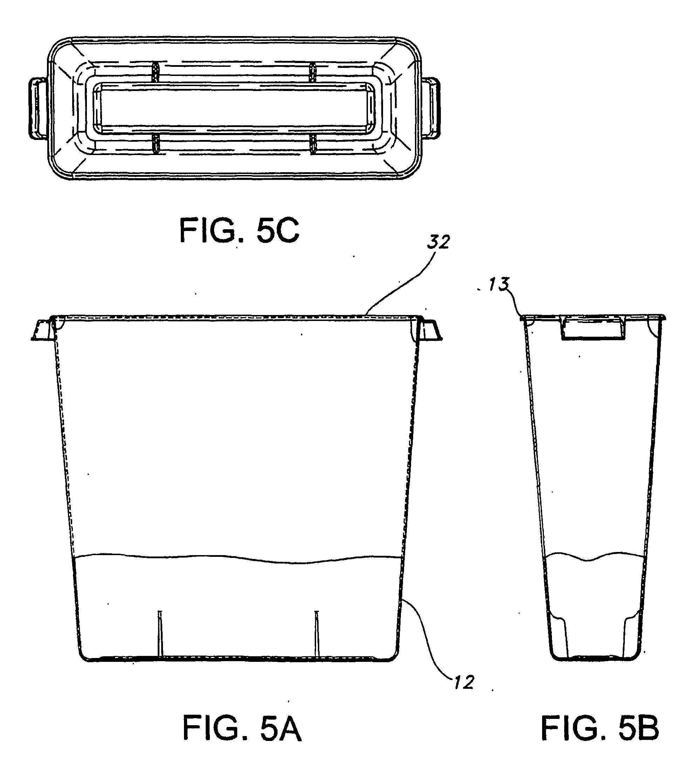

One embodiment of an assembly, with which a closure according to this invention can be used, is illustrated in Fig. 1. A medical waste receptacle 12 defines an opening 32 at the top of the receptacle 12 (as illustrated in Fig. 5A). A closure 10 is mounted on top of the medical waste receptacle 12 at its opening 32. The closure 10 consists of an assembly of a lid component 26, a hood component 14, and a door component 20. Together, the lid 26, hood 14, and door 20 serve to permit the introduction of medical waste such as sharps into the receptacle 12, while preventing access by a user of the assembly to the interior of the receptacle 12.

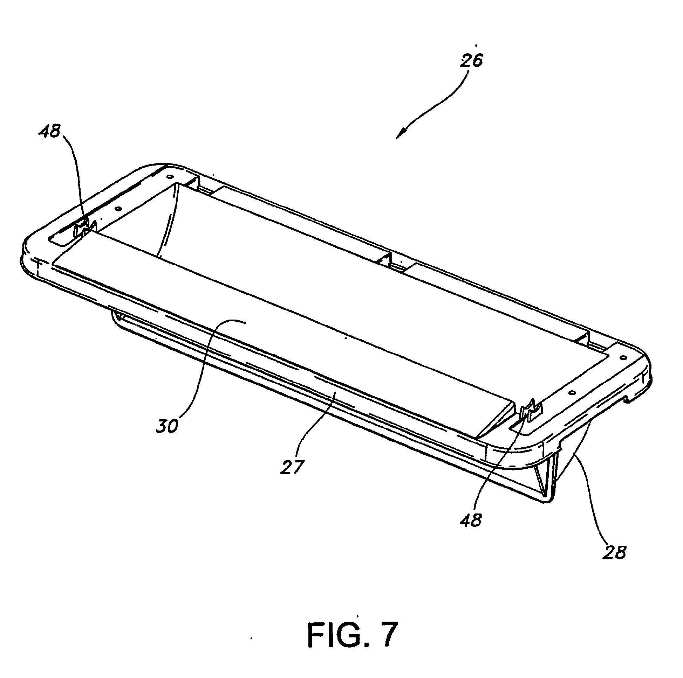

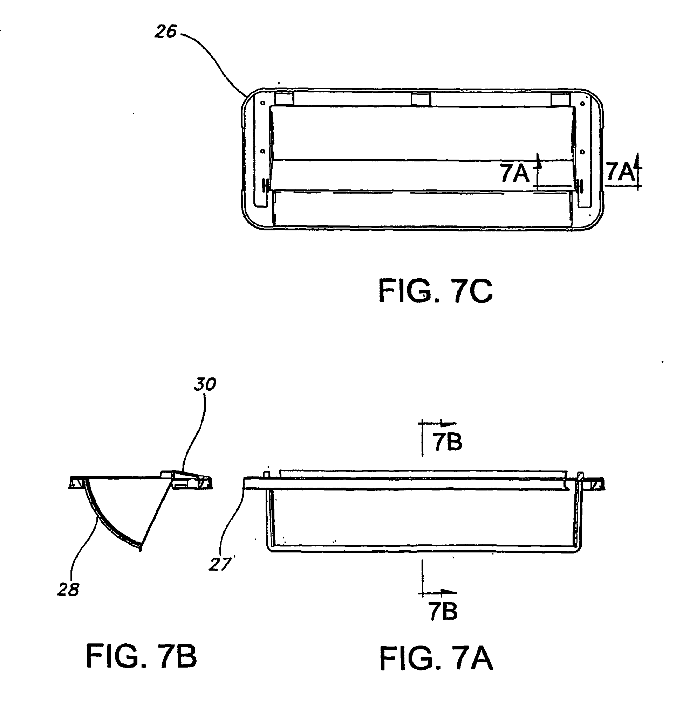

The lid 26 includes a skirt portion 27 (as illustrated in Fig. 7) positioned to engage a lip portion 13 of the receptacle 12 (as illustrated in Fig. 5A), thereby preventing inadvertent removal of the lid 26 from the receptacle 12. The lid 26 also includes a contoured portion 28, sometimes referred to as a cowl, for guiding medical waste into the receptacle 12 from the interior of the hood 14. The lid 26 further includes a ramp portion 30, and provides an overall base structure for the closure 10 assembly.

The door 20 is mounted at least partially within the interior of the hood 14 for movement about a pivot axis 22. The door 20 includes a substantially planar support surface 24 along which medical waste is guided as it is introduced into the assembly. As the door 20 is rotated about pivot axis 22, the increasing incline angle of the support surface 24 will cause the medical waste to travel toward the rear of the container and toward the receptacle 12. As described previously, an access opening 18 is defined by surfaces of the door 20 and the hood 14. Specifically, the door 20 provides a door limiting surface 17, which defines a lower limit to the access opening 18.

The door 20 also includes a series of weight fins 21 positioned along the underside of the door limiting surface 17 that serve to bias the door 20 by gravity toward an opened position, as illustrated in Fig. 1. In other words, because the door 20 is pivotally mounted, the weight of its fins 21 tends to balance the door 20 toward its opened position. By providing a series of fins 21 with a size and quantity sufficient to alter the center of gravity of door 20 with respect to the pivot axis 22, it has been discovered that the access angle "A" of the door 20 in the opened position can be increased. More specifically, by virtue of the weight of material in fins 21, the center of gravity of the door 20 is shifted such that the door 20 can be rotated beyond a vertical position to the closed position, yet return to the opened position when released.

It has been discovered that, by increasing the angle "B" of the door in the closed position (as illustrated in Fig. 4), the size of the disposal opening 29 defined between the door 20 and the lid contoured portion 28 (illustrated in Fig. 3) can be increased. By increasing the size of the disposal opening 29, the assembly can accommodate larger medical waste such as sharps having a larger diameter or thickness.

This ability to accommodate larger sharps (by virtue of the increased angle "B" and the resulting increase in disposal opening 29) can therefore be accomplished without the need for increasing the profile "P" of the assembly (illustrated in Fig. 3). Also, this ability to accommodate larger sharps can be accomplished without the need to significantly alter the lid contoured portion 28 (e.g., by reducing the degree to which it extends toward the front of the assembly). The lid contoured portion 28 is provided, at least in part, to provide a barrier preventing access to the interior of the receptacle 12. It is, therefore, advantageous to avoid undue reduction of the contoured portion 28 as the sole means for increasing the disposal opening 29. The optional increase in angle "B" of door 20 in the closed position, alone or in combination with other exemplary aspects of this invention, therefore facilitates this exemplary feature.

Fig. 2 is a detailed view of the closure 10 in its opened position. The door limiting surface 17 and the hood limiting surface 16 define the access opening 18, and form the access angle "A" defined by the pivot axis 22 of the door 20 and the access opening 18, as illustrated in Fig. 2. The access angle "A" is at least about 60°, which is sufficiently large so that the access opening 18 may accept a wide range of sharp sizes. Preferably, access angle "A" is at least about 80°, and most preferably at least about 83°.

This relatively large access angle "A," alone or combined with additional features described herein, enables the closure according to this invention to provide a medical waste disposal system that can accommodate large sharps, while maintaining a low profile "P." More specifically, by providing an increased access angle "A," the size of the access opening 18 can be increased or maintained without unduly increasing the profile "P" of the assembly. For example, by increasing the angle "A," the distances between the pivot axis 22 and the door limiting surface 17 and between the pivot axis 22 and the hood limiting surface 16 need not be increased to provide an increased access opening 18. While those distances could be increased according to this invention to increase the size of the access opening 18 (e.g., to accommodate larger waste), such an increase could result in an increase in the profile "P" of the assembly, the height of the hood 14, and/or the degree to which the outer edge of the door 20 extends outwardly from the hood 14 in its opened position.

Fig. 3 illustrates the closure 10 in its closed position, and Fig. 4 is a detailed view of the closure 10 in its closed position. The door 20 in this position substantially prevents access to the medical waste receptacle 12 through the access opening 18 of the hood 14. The support surface 24 of the door 20 in the closed position forms an angle "B" with respect to a horizontal plane, as discussed previously. The angle "B" is at least about 100°, and more preferably at least about 105°, and is sufficiently large so that the support surface 24 expands the disposal opening 29 as the door 20 is rotated toward its closed position, thereby permitting a wide range of sharp sizes to drop into the medical waste receptacle 12. It is this relatively large angle "B," alone or combined with an increased access angle "A" described previously and/or other exemplary features of this invention, that enables the closure according to this invention to provide a medical waste disposal system that can accommodate larger sharps, while maintaining a low profile "P."

Referring to Figs. 1-4, the door 20 is biased by gravity, due to the series of weight fins 21 configured along the underside of the door limiting surface 17, to rest naturally toward its opened position, as illustrated in Fig. 1. When a sharps is placed on the support surface 24 of the door 20, it slides or rolls down the support surface 24 causing the door 20 to rotate. The door 20 rotates backwards about its pivot axis 22 toward the closed position illustrated in Fig. 3, and the sharps is deposited into the medical waste receptacle 12. The contoured portion 28 of the lid 26 deflects the sharps toward the medical waste receptacle 12.

Because the door 20 is biased by gravity, it then automatically rotates back forward toward its opened position, illustrated in Fig. 1. Once the medical waste receptacle 12 is full, the user then fastens flexible recessed locking tabs 34 on the hood 14 by pushing the door 20 further into the hood 14, thereby securing the closed door 20 to the hood 14.

The ramp 30 positioned on the lid 26 generally discourages users from placing sharps on the lid of a medical waste disposal system filled to capacity. Although the ramp 30 serves some utility, it is recognized that the configuration of the ramp 30 can take a wide variety of shapes and forms and that the configuration of the ramp 30 illustrated in the figures is primarily an ornamental design, and other configurations are of course contemplated. For example, the ramp 30 can be provided by one or more fins extending upwardly from the surface of the lid 26, by a combination of such fins or ribs with an inclined surface, or by any structure capable of discouraging users from positioning sharps on the lid. Ornamental features of the closure 10 are described in U.S. Design Patent No. D478664 to Moats et al..

Figs. 5A-5C illustrate the medical waste receptacle 12 of Figs. 1 and 3 in detail. The lip 13 of the medical waste receptacle 12 defines a receptacle opening 32 positioned to receive medical waste, and is typically formed from plastic. Further details of a suitable receptacle are disclosed in U.S. Patent No. 5,947,285 to Gaba et al..

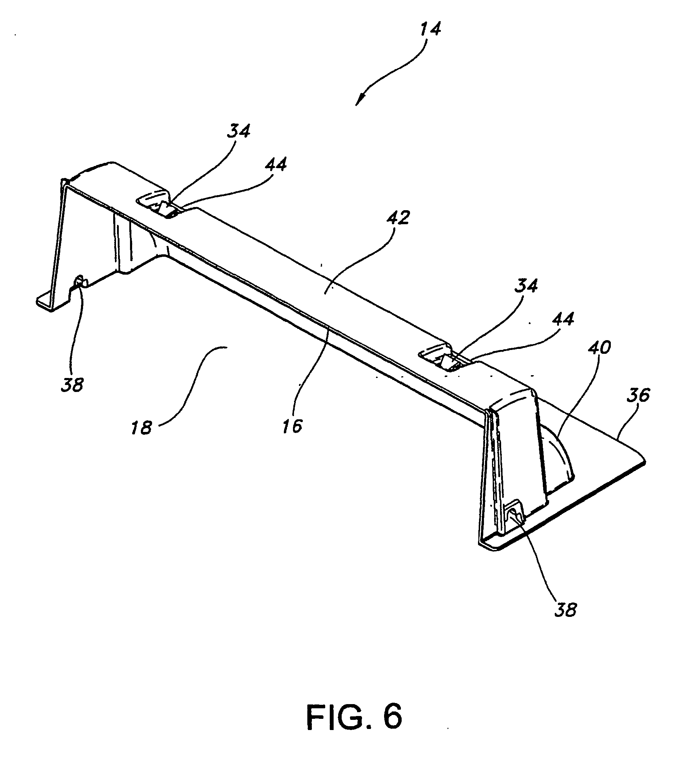

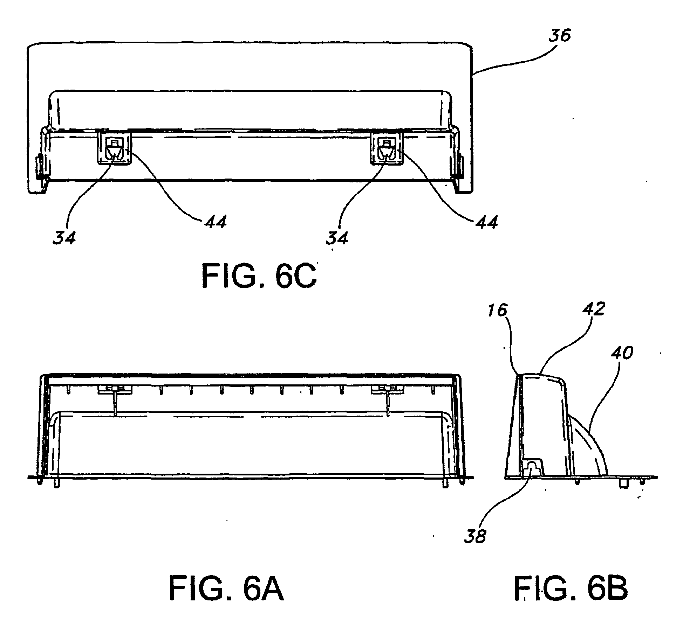

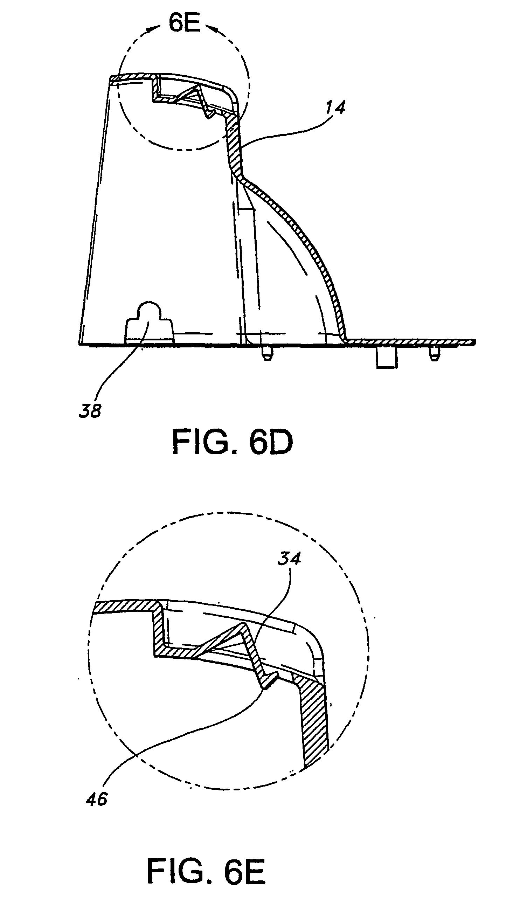

Figs. 6 and 6A-6E illustrate the hood 14 of Figs. 1-4 in detail. The hood 14 includes a hood base 36 for connection with the lid 26. The base 36 defines pivot axis apertures 38 that, in combination with lid pivot axis supports 48 (illustrated in Fig. 7 and described subsequently), house the pivot axis 22 of the door 20. A contoured portion 40 toward the back of the hood 14 permits passage of a range of sharps sizes as they are deposited into the medical waste receptacle 12. A top surface 42 of the hood 14 includes the hood limiting surface 16 which defines the access opening 18 to accommodate a wide range of sharp sizes. The top surface 42 also defines recessed portions 44, which house the recessed locking tabs 34. When the medical waste receptacle 12 is full and the door 20 is in its closed position, the user then depresses the flexible locking tabs 34 into locking tab apertures 50 (illustrated in Fig. 8 and described subsequently), and a hook portion 46 of each locking tab 34 engages the door 20, permanently securing the closed door 20 to the hood 14. Alternatively, the locking tabs 34 are configured to engage the door 20 as the door is urged by the user further into the hood 14, thereby permitting the hook portions 46 of the biased locking tabs 34 to enter corresponding apertures 50 in the door 20, and thereby facilitating engagement between the door 20 and the hood 14. The locking tabs 34 are positioned on the hood 14 in recessed portions 44 to prevent unintentional locking of the door 20. The hood 14 is typically formed from plastic.

The hood 14 can be provided with a wide variety of configurations while still accomplishing the foregoing functions. For example, the shapes of the hood contoured portion 40, the hood top surface 42, the hood limiting surface 42, the recessed portions 44, the hood base 36, and other surfaces of the hood 14 can be modified while still accommodating the door 20, medical waste, and the locking features described herein. The configuration of hood 14 selected for illustration in the figures is dictated by ornamental considerations, and those ornamental aspects of the hood 14 are disclosed in U.S. Design Patent No. D478664 to Moats et al..

Figs. 7 and 7A-7C illustrate the lid 26 in detail. The lid 26 provides an overall base structure for the closure 10 assembly. The lid 26 includes a skirt portion 27 positioned to engage the lip portion 13 of the receptacle 12, thereby preventing inadvertent removal of the lid 26 from the receptacle 12. The lid 26 also includes a contoured portion 28 which, as described previously, deflects sharps toward the medical waste receptacle 12 when sharps are deposited. Furthermore, the lid 26 includes pivot axis supports 48 that support the pivot axis 22 of the door 20. As described previously, the pivot axis apertures 38 of the hood base 36 combine with the lid pivot axis supports 48 to house the pivot axis 22 of the door 20. As described previously, the ramp 30 positioned on the lid 26 discourages users from placing sharps on the lid of a medical waste disposal system filled to capacity. However, it is recognized that the ramp 30 is primarily an ornamental design, and other configurations are of course contemplated. Ornamental features of the lid 26 are further described in U.S. Design patent No. D478664 to Moats et al. The lid 26 is typically formed from plastic.

Figs. 8 and 8A-8C illustrate the door 20 in detail. The door 20 is substantially planar, and is mounted for movement about its pivot axis 22. The door 20 includes a support surface 24 and a door limiting surface 17. The door limiting surface 17 and the hood limiting surface 16 together define the access opening 18 (as illustrated in Fig. 1). The door 20 also includes a series of weight fins 21 configured along the underside of the door limiting surface 17. As described previously, the weight fins 21 serve to bias the door 20 by gravity toward an opened position (as illustrated in Fig. 1).

The support surface 24 of the door 20 is defined by a width "W" and a depth "D." The width to depth ratio "W:D" is at least about 1.9:1, more preferably at least about 2.0:1, and most preferably at least about 2.2:1, and is sufficiently proportioned so that the support surface 24 may accept a wide range of sharp sizes, while maintaining a relatively small depth "D" dimension (and thereby permitting a limited profile "P" of the overall assembly). It is this width to depth "W:D" ratio, alone or combined with a selected access angle "A" and/or a selected rotation angle "B," both described previously, that enables the closure according to this invention to provide a medical waste disposal system that can accommodate large sharps, while maintaining a low profile "P."

The door includes locking tab apertures 50 for engagement with the recessed locking tabs 34 positioned on the hood 14 when the closed door 20 is to be secured. As described previously, when the medical waste receptacle 12 is full and the door 20 is in its closed position, the user depresses the flexible locking tabs 34 into the locking tab apertures 50, and a hook portion 46 of each locking tab 34 engages the door 20, permanently securing the closed door 20 to the hood 14. Alternatively, the hood 14 and door 20 are engaged by virtue of the bias of hook portions 46 toward the locking tab apertures 50 when the door 20 is urged by the user into the hood 14. The door 20 is typically formed from plastic.

Although exemplary embodiments of a closure according to this invention have been described, there are others that support the invention and are therefore within the contemplated scope of the invention. For example, with respect to the access angle "A," it is not limited to at least about 60°, and may be at least about 80° or may be less than 60°. Similarly, the angle "B" is not limited to at least about 100°, and may be at least about 105° or less than 100°. Additionally, the width to depth ratio "W:D" of the door 20 is not limited to at least about 1.9: 1, and may be at least about 2.2: 1 or may be less than 1.9: 1.

Also, it will be appreciated that various exemplary features of the invention can be employed alone or in combination to achieve the objectives of the invention. For example, the increased width:depth ratio "W:D," increased access angle "A," and increased rotation angle "B" can be employed independently or in any combination in order to achieve the objective of increasing the range of sharps sizes that can be accommodated by the assembly without unduly increasing the profile "P" of the assembly. While the exemplary assembly illustrated in the figures employs a combination of all of these features, the features alone or in other combinations can be selected.

It will be appreciated that other modifications can be made to the illustrated embodiment of the closure according to this invention without departing from the scope of the invention. The scope of the invention is separately defined in the appended claims.

## Claims

1. A closure (10) for a medical waste receptacle (12) defining a receptacle opening (32) positioned to receive medical waste, said closure (10) comprising:
a hood (14) positionable adjacent the receptacle opening (32) of the medical waste receptacle (12), said hood (14) having a limiting surface (17) at least partially defining an access opening (18) facilitating access to an interior of said hood (14); and
a door (20) mounted at least partially within said interior of said hood (14), said door (20) being mounted for movement about a pivot axis (22) between an opened position wherein a support surface (24) of said door (20) substantially permits access through said access opening (18) of said hood (14) and a closed position wherein said support surface (24) of said door (20) substantially prevents access through said access opening (18) of said hood (14);
said door (20) being biased by gravity to rotate from said closed position toward said opened position, **characterized in that** said support surface (24) of said door (20) in said closed position forms an angle (B) at least about 100° with respect to a horizontal plane;
said door (20) has a width (W) and a depth (D) defining said support surface (24), said door having a width: depth (W:D) ratio at least about 1.9:1; and
said door (20) and said hood (14) together define said access opening (18) in said opened position and form an access angle (A) defined by said pivot axis (22) of said door (20) and said access opening (18), said access angle (A) being at least about 60°.

2. The closure (10) recited in claim 1, wherein said support surface (24) of said door (20) in said closed position forms an angle (B) at least about 105° with respect to a horizontal plane.

3. The closure (10) recited in claim 1 or claim 2, wherein said door (20) has a width: depth (W:D) ratio at least about 2.2:1.

4. The closure (10) recited in any one of the preceding claims, wherein said access angle (A) is at least about 80°.

5. The closure (10) recited in any one of the preceding claims, wherein said door (20) is substantially planar.

6. The closure (10) recited in claim 5, wherein the plane of said door (20) traverses through said pivot axis (22).

7. The closure (10) recited in any one of claims 1 to 6, wherein said door (20) rotates backwards from said opened position to said closed position.

8. The closure (10) recited in any one of claims 1 to 6, wherein said door (20) rotates forward from said closed position to said opened position.

9. The closure (10) recited in any one of claims 1 to 6, wherein said pivot axis (22) of said door (20) is a vertex of said access angle (A).

10. The closure (10) recited in any one of claims 1 to 6, wherein said closure (10) further comprises a lid (26) positionable adjacent said hood (14), said hood (14) being supported by said lid (26).

11. The closure (10) recited in claim 10, wherein said lid (26) has a portion (28) contoured to deflect medical waste toward the medical waste receptacle (12).

## Patentansprüche

1. Verschluss (10) für einen medizinischen Abfallbehälter (12), der eine Behälteröffnung (32) definiert, die derart angeordnet ist, um medizinische Abfälle aufzunehmen, mit einer anschließend an die Behälteröffnung (32) des medizinischen Abfallbehälters (12) positionierbaren Haube (14), die eine Begrenzungsfläche (17) aufweist, die zumindest teilweise eine Zugangsöffnung (18) definiert, die den Zugang zu einem Innenraum der Haube (14) erleichtert und mit einer Tür (20), die zumindest teilweise innerhalb des Innenraums der Haube (14) befestigt ist, wobei die Tür (20) derart montiert ist, dass sie um eine Schwenkachse (22) zwischen einer geöffneten Position in der eine Auflagefläche (24) der Tür (20) im Wesentlichen Zugang durch die Zugangsöffnung (18) der Haube (14) erlaubt und einer geschlossenen Position, in der die Auflagefläche (24) der Tür (20) im Wesentlichen den Zugang durch die Zugangsöffnung (18) der Haube (14) verhindert, wobei die Tür durch die Erdanziehungskraft vorgespannt ist, um von der geschlossenen Position in die geöffnete Position zu drehen, **dadurch gekennzeichnet, dass** die Auflagefläche (24) der Tür (20) in der geschlossenen Position einen Winkel (B) von zumindest 100° bezüglich einer Horizontalebene bildet, wobei die Tür (20) eine Breite (W) und eine Tiefe (D) hat, die die Auflagefläche (24) definieren, wobei die Tür ein Breite:Tiefe-Verhältnis (W:D) von zumindest etwa 1,9:1 hat und die Tür (20) und die Haube (14) in der geöffneten Position zusammen die Zugangsöffnung (18) definieren und einen von der Schwenkachse (22) der Tür (20) und der Zugangsöffnung (18) definierten Zugangswinkel (A) bilden, der zumindest etwa 60° beträgt.

2. Verschluss (10) nach Anspruch 1, wobei die Auflagefläche (24) der Tür (20) in der geschlossenen Position einen Winkel (B) von zumindest etwa 105° bezüglich zu einer Horizontalebene ausbildet.

3. Verschluss (10) nach einem der Ansprüche 1 oder 2, wobei die Tür (20) ein Breite:Tiefe-Verhältnis (W:D) von zumindest etwa 2,2:1 hat.

4. Verschluss (10) nach einem der vorhergehenden Ansprüche, wobei der Zugangswinkel (A) zumindest etwa 80° beträgt.

5. Verschluss (10) nach einem der vorhergehenden Ansprüche, wobei die Tür (20) im Wesentlichen plan ist.

6. Verschluss (10) nach Anspruch 5, wobei die Ebene der Tür (20) durch die Schwenkachse (22) verläuft.

7. Verschluss (10) nach einem der vorhergehenden Ansprüche, wobei sich die Tür (20) rückwärts von der geöffneten Position in die geschlossene Position dreht.

8. Verschluss (10) nach einem der Ansprüche 1 bis 6, wobei sich die Tür (20) vorwärts von der geschlossenen Position in die geöffnete Position dreht.

9. Verschluss (10) nach einem der vorhergehenden Ansprüche, wobei die Schwenkachse (22) der Tür (20) ein Scheitelpunkt des Zugangswinkels (A) ist.

10. Verschluss (10) nach einem der vorhergehenden Ansprüche, wobei der Verschluss (10) ferner einen Deckel (26) aufweist, der benachbart zu der Haube (14) positionierbar ist, wobei die Haube (14) von dem Deckel (26) getragen wird.

11. Verschluss (10) nach Anspruch 10, wobei der Deckel (26) einen Abschnitt (28) hat, der konturiert ist, um medizinische Abfälle in Richtung des medizinischen Abfallbehälters (12) abzulenken.

## Revendications

1. Fermeture (10) pour un récipient pour déchets médicaux (12) définissant une ouverture de récipient (32) positionnée pour recevoir des déchets médicaux, la fermeture (10) comprenant :
- un capot (14) positionnable à proximité de l'ouverture de récipient (32) du récipient pour déchets médicaux (12), le capot (14) ayant une surface de limitation (17) définissant au moins partiellement une ouverture d'accès (18) facilitant l'accès à un intérieur du capot (14), et
- une porte (20) montée au moins partiellement dans l'intérieur du capot (14), la porte (20) étant montée pour se mouvoir autour d'un axe de pivotement (22) entre une position ouverte où une surface de support (24) de la porte (20) permet largement l'accès à travers l'ouverture d'accès (18) du capot (14) et une position fermée où la surface de support (24) de la porte (20) empêche largement l'accès à travers l'ouverture d'accès (18) du capot (14),
la porte (20) étant décentrée par gravité pour pivoter de la position fermée à la position ouverte,
**caractérisée en ce que**,
dans la position fermée, la surface de support (24) de la porte (20) forme un angle (B) d'au moins environ 100° par rapport à un plan horizontal;
la porte (20) a une largeur (W) et une profondeur (D) définissant la surface de support (24), la porte ayant un rapport largeur/profondeur (W:D) d'au moins environ 1,9/1; et
la porte (20) et le capot (14) définissent ensemble l'ouverture d'accès (18) dans la position ouverte et forment un angle d'accès (A) défini par l'axe de pivotement (22) de la porte (20) et l'ouverture d'accès (18), l'angle d'accès (A) étant d'au moins environ 60°.

2. Fermeture (10) selon la revendication 1,
**caractérisée en ce que**
la surface de support (24) de la porte (20) forme en position fermée un angle (B) d'au moins environ 105° par rapport à un plan horizontal.

3. Fermeture (10) selon la revendication 1 ou la revendication 2,
**caractérisée en ce que**
la porte (20) a un rapport largeur/profondeur (W:D) d'au moins environ 2,2/1.

4. Fermeture (10) selon l'une des revendications précédentes,
**caractérisée en ce que**
l'angle d'accès (A) est d'au moins environ 80°.

5. Fermeture (10) selon l'une des revendications précédentes,
**caractérisée en ce que**
la porte (20) est sensiblement plane.

6. Fermeture (10) selon la revendication 5,
**caractérisée en ce que**
le plan de la porte (20) traverse l'axe de pivotement (22).

7. Fermeture (10) selon l'une des revendications 1 à 6,
**caractérisée en ce que**
la porte (20) pivote vers l'arrière de la position ouverte à la position fermée.

8. Fermeture (10) selon l'une des revendications 1 à 6,
**caractérisée en ce que**
la porte (20) pivote vers l'avant, de la position fermée à la position ouverte.

9. Fermeture (10) selon l'une des revendications 1 à 6,
**caractérisée en ce que**
l'axe de pivotement (22) de la porte (20) est un sommet de l'angle d'accès (A).

10. Fermeture (10) selon l'une des revendications 1 à 6,
**caractérisée en ce que**
la fermeture (10) comprenant en outre un couvercle (26) positionnable à proximité du capot (14), le capot (14) étant supporté par le couvercle (26).

11. Fermeture (10) selon la revendication 10,
**caractérisée en ce que**
le couvercle (26) a une partie (28) profilée pour dévier les déchets médicaux vers le récipient pour déchets médicaux (12).
